# EUROPEAN PATENT APPLICATION

(11) **EP 4 847 031 A1**
(43) Date of publication of application: **02.12.2026**
(21) Application number: 26181428.9
(22) Date of filing: 27.05.2026
(51) Int. Cl.: A61K 36/11, A61K 36/704, A61K 36/752, A61P 13/04

(54) **PHYTOTHERAPEUTIC COMPOSITION FOR THE TREATMENT AND PROPHYLAXIS OF RENAL-URETAL CALCULOSIS**

(30) Priority: 29.05.2025 IT 202500012550
(71) Applicant: Lithos Alb + S.r.l., 33170 Pordenone (IT)
(72) Inventor: LENIQI, Adelajde, Pordenone (IT); RUGGERA, Lorenzo, Pordenone (IT)
(74) Representative: Giugni, Diego

(57) **Abstract**

The present invention relates to a phytotherapeutic composition for the treatment and prophylaxis of reno-ureteral stone disease. In particular, the invention relates to a composition comprising extracts or whole plant parts having marked properties for preventing and disintegrating microlithiasic aggregates responsible for the formation of urinary stones.

## Description

### TECHNICAL FIELD OF THE INVENTION

The present invention relates to a phytotherapeutic composition for the treatment and prophylaxis of reno-ureteral urolithiasis. In particular, the invention relates to a phytotherapeutic composition comprising extracts or whole parts of plants having marked properties capable of inhibiting the crystalline aggregation leading to the formation of urinary stones and promoting the disintegration of such mineral deposits.

### PRIOR ART

Renal lithiasis (also referred to as nephrolithiasis or urolithiasis) is a pathological condition caused by the precipitation, within the excretory urinary tract, of poorly soluble urinary constituents, resulting in the formation of crystalline concretions of varying size.

Although urinary stone disease has existed since ancient times, it remains a highly relevant pathology with significant economic consequences, estimated in the United States at a cost of approximately 2 billion dollars per year.

Despite the considerable progress achieved in medicine over recent decades, a continuous increase in both the prevalence and incidence of urolithiasis has been observed in Western Europe and the United States, where it is estimated that approximately 5% of women and 12% of men will experience a lithiasic episode during their lifetime. Likewise, Italy has also recorded a continuous increase in the incidence and prevalence of nephrolithiasis in recent years and, to date, it is estimated that primary or recurrent urinary stone disease affects approximately 250,000 individuals annually, of whom 40,000 will require one or more treatments (extracorporeal shock wave lithotripsy, endourological procedures, or surgery) for stone clearance.
This increasing trend in the incidence and prevalence of urolithiasis in industrialized countries has been associated with modern lifestyles and dietary habits characterized by a diet rich in sodium, proteins and simple sugars and poor in fiber and alkalis. In the absence of adequate prophylaxis, the recurrence rate of urinary stone disease increases proportionally over time, from 15% after one year to approximately 50% after ten years.

Urinary stones consist of crystalline mineral deposits formed within the renal pyelocaliceal cavities through the aggregation of microscopic crystals. These deposits result from the phase transition of the salts composing them in urine, from the liquid state to the solid state. These deposits result from the phase transition, within the urine, of the salts that compose them, from the liquid state to the solid state. All these changes are induced by the phenomenon of supersaturation, which usually depends on their concentration in the urine and their solubility. If the solubility value is less than 1, the crystals will dissolve; conversely, they will tend to aggregate and grow if such value is greater than 1. A significant contribution to this process is also made by the deficiency of substances that inhibit crystallization and crystal aggregation, such as citrate, magnesium and certain glycoproteins, including Tamm-Horsfall mucoprotein, osteopontin and nephrocalcin.

The composition of urinary stones correlates with the type and degree of urine saturation in the patient. Consequently, together with urinary volume, urinary calcium and oxalate concentrations are the major determinants of calcium oxalate supersaturation; urinary calcium concentration and pH are the major determinants of calcium phosphate supersaturation; whereas urinary pH alone is the major determinant of uric acid supersaturation.

Among these, the most common lithiasic compositions are undoubtedly calcium oxalate and calcium phosphate, which together account for approximately 80% of all urinary stones. It should be emphasized that, in turn, 70-80% of calcium stone disease cases do not present specific metabolic causes and are therefore classified as "idiopathic". In any case, stone formation processes are complex and not yet fully understood; however, as noted above, abnormal urinary pH and abnormal calcium excretion rates appear to be the factors playing a key role in stone pathogenesis in so-called "stone formers", namely individuals at high risk of stone formation and recurrence.

General recommendations for prophylaxis include hydration therapy, namely a high daily fluid intake (2.5-3.5 L/day), in order to maintain a 24-hour urine output of approximately 2,000-2,500 ml, together with an appropriate diet low in oxalate, salt (4-5 g/day) and animal proteins (0.8-1.0 g/kg/day).

Current pharmacological prophylaxis includes thiazide diuretics in order to significantly increase daily urine output. Numerous studies have documented their clinical efficacy in preventing calcium stones. However, thiazide therapy may be associated with certain potential complications, including hypotension, diabetes mellitus and hyperuricemia. Furthermore, patients receiving hydrochlorothiazide treatment should undergo regular skin examinations, as they are at increased risk of developing both non-melanoma skin cancers (NMSC - squamous cell carcinoma and basal cell carcinoma) and certain forms of melanoma.

The treatment of urinary stones further involves their removal by means of non-invasive procedures, such as extracorporeal shock wave lithotripsy, or minimally invasive procedures such as ureterorenoscopy, percutaneous lithotripsy and robotic surgery, up to actual open surgical interventions, which fortunately have now become very rare.

Alternatively, for a long time it has been common folk tradition to treat and prevent kidney stones through the use of medicinal plants. Considering the risk of recurrence of urinary stone disease, the high cost of treatments, and the potential complications arising from surgical procedures themselves, the use of medicinal plants may represent a valid alternative in the prevention of nephrolithiasis. Nevertheless, to date there is still no single phytotherapeutic formulation or composition capable of effectively preventing or treating kidney stone disease. In other words, numerous phytotherapeutic products currently on the market have demonstrated more or less significant effects in the prevention of urinary stone disease, mainly related to their diuretic action.

In particular, such activity is known for *Equisetum hyemale* (a species of the genus *Equisetum*, also known as "horsetail") and for *Polygonum aviculare*, also known as "Knotgrass" (a species of the genus *Polygonum*).

The genus *Equisetum*, and specifically the species *hyemale*, is characterized by genitourinary anti-inflammatory activity, as well as known diuretic, bone-mineralizing and antimicrobial/antifungal/antiparasitic effects. However, the aforementioned effects have never been supported by solid scientific data capable of confirming a truly effective preventive or curative activity for urolithiasis. In other words, there are no scientific data demonstrating real efficacy in treatment, particularly in so-called "stone former" patients.

The second plant belongs, as mentioned above, to the genus *Polygonum*, characterized by antioxidant, anti-inflammatory, diuretic, antimicrobial, neurological, antitumor, antidiabetic, antiparasitic, hepatoprotective and gastroprotective activities. In this case as well, the data available in the literature do not confirm any real efficacy in the treatment of stone disease, particularly in "stone former" patients, either with regard to prevention or stone disintegration.

In practice, although the two plants identified above appear potentially interesting for the treatment and prevention of stone disease, there are currently no specific compositions or therapies capable of being truly effective, particularly for the group of patients referred to as "stone formers".

For example, document US2013/0064912A1 describes a nutraceutical formulation based on homeopathic ingredients in combination with traditional herbs and/or nutraceutical ingredients for relieving symtoms associated to kidney stones and to gallstones. The formulation describes the use of *Polygonum* or *Equisetum* in association with *Nux vomica* and/or *Berberis vulgaris*. *Polygonum* and *Equisetum* are indicated for supporting liver and kidney functions, but not specifically for the treatment of kidney stones. Furthermore, no specific therapeutic regimens or treatment protocols for "stone former" patients are described.

The document *"*Home Remedies for Kidney Stones: 12 Teas & Natural Remedies" (https://www.tuasaude.com/en/home-remedies-for-kidney-stones/) describes the use of lemon juice to eliminate kidney stones and delay their growth. It also describes the use of horsetail tea as a diuretic. The use of this treatment for "stone former" patients is not described, and administration of horsetail tea for more than one week is discouraged because of side effects such as dehydration, diarrhea, tachycardia, weight loss, pancreatitis and muscle weakness.

The document *"*Consumption of Historical and Current Phytotherapeutic Agents for Urolithiasis: A Critical Review" (Gurocak et al., Journal of Urology, Elsevier, Vol. 176, No. 2, 1 August 2006, pp. 450-455) describes the state of the art concerning the use of certain plants as potential agents for the treatment of urolithiasis. Despite the potential benefits reported in traditional medicine, no concrete clinical data exist, while known side effects have been reported.

The document *"*Herbal Medicines in the Management of Urolithiasis: Alternative or Complementary?" (Planta Medica, Vol. 75, No. 10, 14 May 2009, pp. 1095-1103) describes the experimental use of certain plants in the treatment of kidney stones. In addition to these plants, lemon juice is cited as an effective component in rats treated with ethylene glycol-ammonium chloride. Neither *Polygonum* nor *Equisetum* is mentioned, nor are any specific therapeutic treatment regimens for "stone former" patients described.

The document *"*Phytochemistry and Pharmacology of the Genus Equisetum (Equisetaceae): A Narrative Review of the Species with Therapeutic Potential for Kidney Disease" (Evidence-Based Complementary and Alternative Medicine, Vol. 2021, 5 March 2021, pp. 1-17) provides an overview of the genus *Equisetum* for use in traditional medicine in various applications, including the treatment of kidney stones. Despite characterization of its chemical composition, there are still no scientific data supporting its efficacy in the treatment of renal disorders. In particular, there are no data demonstrating that this genus of plant is effective in the treatment of kidney stones.

Document WO2019/238254A1 describes a phytopharmaceutical composition for the treatment and prophylaxis of, among other conditions, kidney stones, comprising *Equisetum arvense* at a concentration of 3%-67% by weight, *Spergularia rubra* at a concentration of 2%-38% by weight, *Peumus boldus* at a concentration of 1.5%-32% by weight, *Opuntia ficus-indica* at a concentration of 1%-20% by weight, *Sideritis angustifolia* at a concentration of 1%-20% by weight, *Rosmarinus officinalis* at a concentration of 1%-20% by weight, *Cynodon dactylon* at a concentration of 1%-20% by weight, and *Melissa officinalis* at a concentration of 1%-20% by weight.

The document "Herbal Tea for Kidney Stones Kronos - Prevent & Relieve Kidney - Lelex Tea" (6 February 2022, https://lelextea.com/en/greek-organic-herbs-herbal-teas-mountain-tea/herbal-tea-blend-stone-crusher-kronos/) describes a composition comprising Bermuda grass, rosehip, bearberry, St. John's wort, spearmint, olive (Olea L.), corn silk, cherry stalks, rock rose, fennel seeds, parsley, broadleaf plantain, horsetail, birch, rustyback fern (Asplenium), dandelion and nettle. These herbs are used as an infusion to improve kidney health and help prevent and facilitate the passage of kidney stones. No specific treatment regimen is described, particularly for "stone former" patients, nor is any preventive or stone-disintegrating action on kidney stones disclosed.

The document "Research Landscape and Pharmacological Mechanisms of Traditional Chinese Medicines in Treating and Preventing Urolithiasis: Unearthing an Anti-Urolithic Treasure Trove" (Jiang Qinhong et al., Journal of Ethnopharmacology, Elsevier, Vol. 334, 29 June 2024) describes the state of the art of traditional Chinese medicine in the treatment of urolithiasis. In particular, the inhibition of lithogenesis by Polygonum cuspidatum is described. However, no specific dosage regimen is disclosed.

Document CN118340859 describes compositions according to traditional Chinese medicine for the treatment of kidney stones. The compositions comprise the following raw materials, in parts by weight:
35-45 parts Herba desmodii styracifolii,
25-35 parts Lygodium japonicum,
25-35 parts aromatic turmeric,
8-12 parts raw tuber of Gallus gallus,
12-18 parts Pyrrosia ovata,
12-18 parts talc,
10-15 parts raw Rehmannia root,
10-15 parts Polygonum aviculare,
12-18 parts Plantago asiatica seeds,
12-18 parts aniseed,
8-12 parts Cyathula officinalis,
25-35 parts white peony root,
10-15 parts honey-processed licorice root,
4-8 parts Citrus reticulata pericarp,
25-35 parts raw astragalus,
and 12-18 parts Angelica sinensis.

The document "Medicinal Uses, Phytochemistry, Pharmacology and Taxonomy of Polygonum aviculare L.: A Comprehensive Review" (Benrahou Kaoutar et al., Medicinal Chemistry Research, Birkhäuser, Vol. 32, No. 3, 25 January 2023, pp. 409-423) describes the use of Polygonum aviculare for the treatment of various disorders, including those related to the kidneys. No specific treatment regimens are described, particularly for "stone former" patients.

The document "Start Tea Drink N51" (Mintel; 18 August 2015, GNPD database) describes a tea composed of chamomile, nettle, bearberry, birch, cornflower, knotgrass (Polygonum), mint, lemon balm, common horsetail, corn stigmas, bean leaves, thyme, sunflower root, burdock, couch grass and wild rose root. Among the claimed effects, a contribution to the dissolution of salt deposits is mentioned. No treatment regimen is indicated, particularly for the treatment of kidney stones in "stone former" patients.

### SUMMARY OF THE INVENTION

The technical problem underlying the present invention was therefore to provide a phytotherapeutic composition for the treatment and prevention of urinary stone disease comprising plant extracts that are genuinely effective.

This problem is solved by a combination of plant extracts in specific quantities to be administered according to specific dosage regimens.

A first object of the present invention was therefore to identify a composition of extracts obtained from parts of three specifically selected and combined plants.

A second object was to obtain a composition based on extracts of the three selected plants, specifically intended for administration in the form of an infusion or tea.

A further object is a dosage regimen of a phytotherapeutic composition for the treatment and prevention of urinary stone disease.

### DETAILED DESCRIPTION OF THE INVENTION

As explained in the introductory part of the present description, although the use of horsetail (*Equisetum*) and knotgrass (*Polygonum*) has been suggested in genitourinary infections, there is no reliable scientific evidence demonstrating the actual activity of these two plants, particularly in the subgroup of patients known as "stone formers", i.e., patients especially prone to the formation of calcium oxalate and calcium phosphate kidney stones. This group of patients is particularly susceptible to recurrences, which often require even major surgical treatments.

Starting from the traditional folk use of the two plants separately, it was decided to combine them together and additionally associate them with lemon juice in order to improve their efficacy in the prophylaxis and treatment of lithiasis, particularly in the aforementioned group of stone-former patients. During the initial clinical experimentation, it was surprisingly observed that such a combination proved particularly effective in the treatment and prevention of reno-ureteral stone disease, especially in "stone former" patients, as evidenced by the experimental data reported below in the embodiments of the present invention, in which an unexpected synergistic effect appears.

Furthermore, a specific treatment regimen was identified that proved surprisingly advantageous for therapeutic purposes and free from undesirable side effects.

According to the present invention, a phytotherapeutic composition is proposed comprising a mixture of plant parts of *Equisetum* and *Polygonum* together with lemon juice. In particular, the composition is in the form of tea, infusion, decoction, or juice obtained by cold pressing plant parts.

Preferably, the infusion comprises, per 1000 ml of water, plant parts of *Equisetum* and *Polygonum*, preferably dried, in a weight ratio ranging from 1:2 to 2:1, preferably 1:1 (40 g total comminuted plant material: 20 g of *Equisetum* and 20 g of *Polygonum*), and lemon juice in an amount ranging from 100 ml to 300 ml, preferably 200 ml. In particular, both *Equisetum* and *Polygonum* plant parts are present in amounts ranging from 10 g to 50 g per 1000 ml of water.

*Equisetum* is a plant belonging to the family Equisetaceae (order Equisetales, class Equisetopsida). It is a genus of perennial plants widely distributed throughout the world and adapted to grow in temperate, tropical and cold regions. Commonly known as "horsetail", *Equisetum* is, from a phylogenetic standpoint, a very primitive plant lacking distinct sexual organs and reproducing by means of spores. To date, approximately 30 species of *Equisetum* are known, widely distributed throughout Europe, the American continent, and northeastern Asia as far as China, while absent from Australia, New Zealand and Antarctica. The most common and well-known species include *Equisetum hyemale* L., *E. arvense* L., *E. telmateia* Ehrh., *E. giganteum* L., *E. bogotense* Kunth, *E. debile* Roxb. ex Vaucher, *E. myriochaetum*, *E. fluviatile* L., *E. palustre* L., *E. sylvaticum* L., *E. laevigatum*, and *E. ramosissimum* Desf. Among these species, *Equisetum hyemale* and *Equisetum arvense* are preferred, with *Equisetum hyemale* being the most preferred.

*E. hyemale* frequently grows along riverbanks and streams, in shallow water where its stem develops. It appears as a shrub very similar in appearance to bamboo canes, although much smaller in size, capable of reaching two meters in height. It is characterized by hollow, fragile, unbranched cylindrical stems of dark green color, with spaced rings surrounding the stem and emerging from the joints. It is characterized by hollow, fragile, unbranched cylindrical stems of dark green color, with spaced rings surrounding the stem and emerging from the joints.

The plant parts of *Equisetum* used for the infusion, tea, decoction or juice according to the present invention are the aerial stems and/or the needle-like leaves. For the preparation of the infusion, tea or decoction according to the present invention, the above-mentioned plant parts are preferably dried and crushed (comminuted plant material). Subsequently, they are boiled in water for a period ranging from 20 minutes to 50 minutes, preferably 30 minutes.

This procedure also allows the inactivation of the "thiaminase" contained in *Equisetum*, an enzyme that breaks down thiamine (vitamin B1). This thermolabile enzyme is completely inactivated at 80°C, with approximately 50% denaturation occurring at 70°C.

Thereafter, the water is filtered to remove the solid portion of the infusion. Alternatively, the comminuted plant parts may be contained in a suitable bag of the type commonly used for tea or herbal infusions, either disposable or reusable.

Preferably, for every 1000 ml of water to be boiled, from 10 g to 50 g of *Equisetum* plant parts are used, more preferably from 15 g to 30 g, and even more preferably 20 g.

The genus *Polygonum*, belonging to the family Polygonaceae (order: Polygonales; class: Dicotyledoneae), comprises more than 300 species distributed worldwide. This genus includes annual and perennial plants that thrive in a variety of climatic zones, ranging from northern temperate regions to tropical and subtropical areas. The various species are widely distributed throughout Europe, North Africa, Asia, and the Americas (North and South America), and are frequently found along slopes, roadsides, and cultivated areas.
From a morphological and botanical perspective, the genus *Polygonum* varies considerably from one species to another, ranging from prostrate annual herbaceous plants reaching only 5 cm in height, to erect perennial herbaceous plants between 3 and 4 meters high, and even perennial woody vines capable of climbing trees and reaching heights of 20-30 meters.

Numerous species belong to the genus *Polygonum*, among which the best known and most widely used are *Polygonum aviculare*, *P. barbatum*, *P. bellardii*, *P. bistorta* L., *P. capitatum*, *P. cognatum*, *P. equisetiforme*, *P. flaccidum*, *P. glabrum*, *P. multiflorum* Thunb., *P. orientale* Linn., *P. polystachyum*, *P. plebeium*, *P. tortuosum*, *P. viviparum*, and *P. hyrcanicum*. Among these species, *Polygonum aviculare* is preferred.

Based on the experimental results obtained using the composition of the present invention, it appears that *Polygonum aviculare* is particularly effective due to the presence of compounds such as saponins, phenolic substances and flavonoids, which may exert a fat-reducing effect. A diet rich in fats, in fact, may lead to various disorders, including hyperoxaluria, hypercalciuria and nephrocalcinosis.
Furthermore, its probable antioxidant activity may reduce the release of free radicals, which appear to promote stone formation by inducing the heterogeneous nucleation of calcium oxalate crystals and their subsequent aggregation. Furthermore, its probable antioxidant activity may reduce the release of free radicals, which appear to promote stone formation by inducing the heterogeneous nucleation of calcium oxalate crystals and their aggregation.

The parts of *Polygonum* used for the infusion, tea, decoction or juice according to the present invention consist of the entire plant (leaves, flowers, stems and roots). For the preparation of the infusion, tea or decoction according to the present invention, the above-mentioned plant parts are dried and crushed. They are then boiled in water for a period ranging from 20 minutes to 50 minutes, preferably 30 minutes. Subsequently, the water is filtered to remove the solid portion of the infusion. Alternatively, the plant parts may be placed in a suitable bag of the type commonly used for tea or herbal infusions, either disposable or reusable.

Preferably, for every 1000 ml of water to be boiled, from 10 g to 50 g of *Polygonum* plant parts are used, more preferably from 15 g to 30 g, and even more preferably 20 g.

The lemon juice is obtained by simply squeezing fresh lemon pulp and is added, in the amounts indicated above, to the infusion containing the aforementioned parts of the two plants.

Preferably, the preparation of the infusion, tea or decoction comprises the steps of:
- bringing 1000 ml of water to a boil;
- adding from 10 g to 50 g of dried and crushed *Equisetum* parts and from 50 g to 10 g of dried and crushed *Polygonum* parts;
- allowing the mixture to infuse for 20-60 minutes while cooling to room temperature;
- filtering the infusion;
- squeezing lemons to obtain from 100 ml to 300 ml of juice to be added to the infusion;
- storing the preparation until consumption in a dry, cool place protected from sunlight, but not in a refrigerator.

According to a second aspect of the present invention, regarding the treatment regimen, the infusion is administered according to a specific protocol providing for intake in amounts of 2.5-10 cl six times daily, before and after the main meals of breakfast, lunch and dinner.
Preferably, 5 cl are administered six times daily, 30 minutes before and 30 minutes after the main meals.
Indeed, it has been observed that this precise protocol provides optimal efficacy both in the prevention and in the complete destruction of kidney stones, or at least of the crystalline formations that subsequently aggregate and lead to their formation.
In particular, when the treatment is carried out in cycles of 1-3 months, separated by intervals of 3-6 months between cycles, complete remission of the disease has been observed, especially in "stone former" patients.

These surprising results have led to the assumption of a synergistic effect arising from the combination of the above-mentioned three herbal components. Furthermore, the therapeutic protocol adopted has confirmed the effective preventive and eliminative action, at least against calcium oxalate and calcium phosphate crystals which, by aggregating, lead to the formation of actual stones.

In this regard, several examples of patients treated with the composition of the present invention are reported below. The cases presented show how the composition of the invention proved effective in reducing and/or eliminating recurrent episodes of reno-ureteral stone disease and, consequently, the need for endoscopic and surgical procedures. Furthermore, it may also contribute to reducing recurrent urinary tract infections, which are often associated with nephrolithiasis (infected stones and infection-induced stone disease).

### EXAMPLE 1

### Patient Data:

### Male, 54 years old

Family history: father and brother with a history of kidney gravel (chemical composition unspecified).

Urological history: since 2002, frequent episodes of bilateral reno-ureteral stone disease with spontaneous passage of small stones. Since 2008, the patient has undergone several endourological treatments, both percutaneous and retrograde transureteral. Recurrences occurred almost annually until 2016.

Stone composition: initially monohydrate calcium oxalate (CaOx); since 2017, dihydrate calcium oxalate.

### Phytotherapy

2017: initiation of therapy with an infusion containing:
- 20 g of *Equisetum hyemale,*
- 20 g of *Polygonum aviculare,*
- in 1000 ml of water,
- plus 200 ml of lemon juice.

### Treatment protocol:

300 ml/day of infusion divided into six administrations of 5 cl each, before and after the main meals (breakfast, lunch and dinner), administered in three-month cycles alternating with three-month periods without therapy.

### Outcome

From 2017 to 2022, during the period of therapy use, the patient reported occasional spontaneous passage of millimetric stones (chemical-physical analysis: always dihydrate calcium oxalate), with prolonged intervals between episodes.

In February 2022, therapy was discontinued.

In December 2022, ultrasound examination revealed recurrent renal stone disease.

In this case, in addition to obtaining an effective reduction in stone formation, it was observed that discontinuation of the treatment according to the invention resulted in recurrence, thereby confirming not only its therapeutic effect but also its preventive efficacy.

### EXAMPLE 2

### Patient Data:

### Female, 28 years old

Family history: mother with a history of kidney stone disease (chemical composition unspecified).

Urological history: 2 cm kidney stone in 2020. In July 2020, ureterorenoscopy was performed; in November 2020, a second RIRS (Retrograde Intrarenal Surgery) was performed to remove residual fragments.

Stone composition: dihydrate calcium oxalate.

### Phytotherapy

Therapy was initiated in 2021 with an infusion containing:
- 20 g of *Equisetum hyemale,*
- 20 g of *Polygonum aviculare,*
- in 1000 ml of water,
- plus 200 ml of lemon juice.

Treatment protocol: 300 ml/day of infusion divided into six administrations of 5 cl each before and after the main meals (breakfast, lunch and dinner), with cyclic repetition of the therapy (2 months) every six months.

### Outcome

Ultrasound follow-up examinations have remained negative for recurrent stone disease to date.

### EXAMPLE 3

### Patient Data:

### Female, 58 years old

Family history: negative for urinary stone disease.

Urological history: bilateral urinary stone disease since 2017, with occasional renal colic and spontaneous passage of yellowish, spiculated, friable stones. The patient underwent two extracorporeal shock wave lithotripsy treatments for left renal lithiasis in 2019. Stone composition: uric acid.

### Phytotherapy

Therapy was initiated in 2021 with an infusion containing:
- 20 g of *Equisetum hyemale,*
- 20 g of *Polygonum aviculare,*
- in 1000 ml of water,
- plus 200 ml of lemon juice.
Treatment protocol: 300 ml/day of infusion divided into six administrations of 5 cl each before and after the main meals (breakfast, lunch and dinner), repeated cyclically every year. Outcome

Subsequent ultrasound examinations have remained negative for renal stone disease to date.

### EXAMPLE 4

### Patient Data:

### Male, 49 years old

Family history: negative for urinary stone disease.

Urological history: patient affected by Crohn's disease, with a history of recurrent left renal stone disease since 2015. The patient underwent several extracorporeal shock wave lithotripsy and transureteral endourological procedures (last left ureterorenoscopy in 09/2022) .

Stone composition: calcium phosphate.

### Phytotherapy

Therapy was initiated in 2020 with an infusion containing:
- 20 g of *Equisetum hyemale,*
- 20 g of *Polygonum aviculare,*
- in 1000 ml of water,
- plus 200 ml of lemon juice.
Treatment protocol: 300 ml/day of infusion divided into six administrations of 5 cl each before and after the main meals (breakfast, lunch and dinner), repeated cyclically every year. Outcome

After initiation of therapy, only two recurrent lithiasic episodes occurred, both on the left side (one ESWL procedure in 2021 and one ureterorenoscopy in September 2022).

### EXAMPLE 5

### Patient Data:

### Male, 53 years old

Family history: negative for urinary stone disease.

Urological history: bilateral reno-ureteral stone disease since 2011, with episodes of hematuria. Over the years, the patient underwent two percutaneous lithotripsies on the left side and three ureteroscopies (two on the right side and one on the left side). The last left-sided URS was performed in 2020.

Stone composition: mixed calcium oxalate (monohydrate and dihydrate).

### Phytotherapy

Therapy was initiated in 2018 with an infusion containing:
- 20 g of *Equisetum hyemale,*
- 20 g of *Polygonum aviculare,*
- in 1000 ml of water,
- plus 200 ml of lemon juice.
Treatment protocol: 300 ml/day of infusion divided into six administrations of 5 cl each before and after the main meals (breakfast, lunch and dinner), used cyclically twice per year. Outcome

Significant reduction in recurrences and, since 2020, absence of new stone formation.

### EXAMPLE 6

### Patient Data:

### Female, 45 years old

Family history: brother with kidney stone disease (chemical composition unspecified).

Urological history: stone disease since 2015, with recurrent episodes of renal colic. Multiple ESWL procedures and ureterorenoscopies were performed, with endoscopic evidence of possible Randall's plaques.

Stone composition: monohydrate calcium oxalate.

### Phytotherapy

Therapy was initiated in 2019 with an infusion containing:
- 20 g of *Equisetum hyemale,*
- 20 g of *Polygonum aviculare,*
- in 1000 ml of water,
- plus 200 ml of lemon juice.

Treatment protocol: 300 ml/day of infusion divided into six administrations of 5 cl each before and after the main meals (breakfast, lunch and dinner), with cyclic therapy lasting 2-3 months every six months.

### Outcome

Since initiation of therapy, a significant increase in the time interval between recurrences has been observed.

### EXAMPLE 7

### Patient Data:

### Male, 67 years old

Family history: negative for urinary stone disease.

Urological history: recurrent right renal stone disease since 2010, with recurring episodes of renal colic (every 2-3 years). Over the years, the patient underwent multiple ESWL procedures and RIRS interventions.

Stone composition: mixed calcium oxalate (monohydrate and dihydrate).

### Phytotherapy

Therapy was initiated in 2016 with an infusion prepared according to the protocol described above, containing:
- 20 g of *Equisetum hyemale,*
- 20 g of *Polygonum aviculare,*
- in 1000 ml of water,
- plus 200 ml of lemon juice.

Treatment protocol: 300 ml/day of infusion divided into six administrations of 5 cl each before and after the main meals (breakfast, lunch and dinner), with cyclic therapy every six months. Outcome

Reduction in recurrence rates and prolonged stone-free intervals.

### EXAMPLE 8

### Patient Data:

### Female, 38 years old

Family history: negative for urinary stone disease.

Urological history: history of left-sided reno-ureteral stone disease since 2018; one ureterorenoscopy in 2018 and another in 2019.

Stone composition: dihydrate calcium oxalate.

### Phytotherapy

Therapy was initiated in 2020 with an infusion prepared according to the protocol described above, containing:
- 20 g of *Equisetum hyemale,*
- 20 g of *Polygonum aviculare,*
- in 1000 ml of water,
- plus 200 ml of lemon juice.

Treatment protocol: 300 ml/day of infusion divided into six administrations of 5 cl each before and after the main meals (breakfast, lunch and dinner), used cyclically every year until 2022. Outcome

Subsequent ultrasound examinations remained negative.

### EXAMPLE 9

### Patient Data:

### Male, 40 years old

Family history: father with a history of stone disease (chemical composition unspecified).

Urological history: recurrent bilateral reno-ureteral stone disease since 2016, with episodes of spontaneous stone passage associated with gross hematuria. In 2018, the patient underwent three RIRS procedures on the right side and one on the left side within the same year (the last in September 2018).

Stone composition: monohydrate calcium oxalate.

### Phytotherapy

Therapy was initiated in 2018 with an infusion containing:
- 20 g of *Equisetum hyemale,*
- 20 g of *Polygonum aviculare,*
- in 1000 ml of water,
- plus 200 ml of lemon juice.

Treatment protocol: 300 ml/day of infusion divided into six administrations of 5 cl each before and after the main meals (breakfast, lunch and dinner), repeated cyclically every six months. Outcome

Since 2019, two additional episodes of right renal colic with spontaneous stone passage (dihydrate calcium oxalate) have been reported, without the need for surgical intervention.

### EXAMPLE 10

### Patient Data:

### Female, 42 years old

Family history: negative for urinary stone disease.

Urological history: left renal stone disease since 2021, stone diameter 2.5 cm; the patient underwent percutaneous lithotripsy in January 2022.

Stone composition: calcium phosphate.

### Phytotherapy

Therapy was initiated in 2022 with an infusion prepared according to the protocol described above, containing:
- 20 g of *Equisetum hyemale,*
- 20 g of *Polygonum aviculare,*
- in 1000 ml of water,
- plus 200 ml of lemon juice.

Treatment protocol: 300 ml/day of infusion divided into six administrations of 5 cl each before and after the main meals (breakfast, lunch and dinner), with cyclic therapy repeated annually. Outcome

Radiological follow-up examinations have remained negative for recurrent stone disease.

### EXAMPLE 11

### Patient Data:

### Male, 70 years old

Family history: negative for urinary stone disease.

Urological history: recurrent bilateral renal stone disease since 2015. The patient underwent multiple bilateral ESWL treatments and retrograde transureteral procedures on the right side. From 2018 to 2022 he remained stone-free. The last right-sided RIRS was performed in 2022.

Stone composition: uric acid.

### Phytotherapy

Therapy was initiated in 2018 with an infusion containing:
- 20 g of *Equisetum hyemale,*
- 20 g of *Polygonum aviculare,*
- in 1000 ml of water,
- plus 200 ml of lemon juice.

Treatment protocol: 300 ml/day of infusion divided into six administrations of 5 cl each before and after the main meals (breakfast, lunch and dinner), used cyclically for two years. Therapy was resumed in 2022 after the last episode of renal stone disease and is still ongoing.

### Outcome

Recurrence of uric acid nephrolithiasis was observed after discontinuation of therapy. Currently, ultrasound follow-up examinations remain negative for recurrent stone disease.

Also in this case, discontinuation of treatment with the composition of the invention demonstrated its preventive and therapeutic efficacy.

### EXAMPLE 12

### Patient Data:

### Male, 30 years old

Family history: negative for urinary stone disease.

Urological history: ultrasound finding of bilateral renal microlithiasis in 2021. No episodes of acute renal colic.

### Phytotherapy

Therapy was initiated in 2022 with an infusion containing:
- 20 g of *Equisetum hyemale,*
- 20 g of *Polygonum aviculare,*
- in 1000 ml of water,
- plus 200 ml of lemon juice.

Treatment protocol: 300 ml/day of infusion divided into six administrations of 5 cl each before and after the main meals (breakfast, lunch and dinner); three treatment cycles of two months each, with a two-month interval between cycles.

### Outcome

Since the start of therapy, no episodes of renal colic or stone passage have been reported.

Regular monitoring with renal ultrasound examinations every six months documented the absence of bilateral renal calcifications.

### EXAMPLE 13

### Patient Data:

### Female, 25 years old

Family history: negative.

Urological history: history of recurrent post-coital urinary tract infections, with frequent episodes over the last five years; no kidney stones or renal gravel.

### Phytotherapy

Therapy was initiated in 2022 with an infusion containing:
- 20 g of *Equisetum hyemale,*
- 20 g of *Polygonum aviculare,*
- in 1000 ml of water,
- plus 200 ml of lemon juice.

Treatment protocol: 300 ml/day of infusion divided into six administrations of 5 cl each before and after the main meals (breakfast, lunch and dinner).

Administration schedule: four-month treatment cycle.

### Outcome

After four months of treatment, the patient reported a significant reduction in infectious episodes, with only one episode occurring over the course of one year.

### EXAMPLE 14

### Patient Data:

### Female, 52 years old

Family history: negative.

Urological history: recurrent urinary tract infections caused by *Proteus mirabilis* over the last three years (>2 episodes/year); right renal microlithiasis documented by ultrasound, with previous spontaneous passage of a millimetric stone (chemical-physical analysis: struvite).

### Phytotherapy

Therapy was initiated in 2023 with an infusion containing:
- 20 g of *Equisetum hyemale,*
- 20 g of *Polygonum aviculare,*
- in 1000 ml of water,
- plus 200 ml of lemon juice.

Treatment protocol: 300 ml/day of infusion divided into six administrations of 5 cl each before and after the main meals (breakfast, lunch and dinner), with three cycles of two months each separated by two-month periods without therapy.

### Outcome

Significant subjective symptomatic improvement was observed. Monitoring every 3-6 months by means of urinalysis and urine culture yielded negative results; semiannual abdominal ultrasound examinations documented the stability of the right renal microlithiasis.

From the foregoing, it is evident that the phytotherapeutic composition of the invention is actually effective compared with any other composition of the prior art. Indeed, although various formulations have been proposed, none has been able to demonstrate systematically that a specific combination of the ingredients of the invention, in terms of defined weight ratios, precise extraction times, controlled addition of lemon juice, and a standardized dosage regimen, possesses clinically documented efficacy in "stone former" patients, either as prophylaxis or as treatment.

In the scientific literature, the plants have been studied separately, and lemon juice is not used as an integral part of a phytotherapeutic formulation. In the present invention, on the other hand, there is a functional and synergistic association of the plants, whose pathophysiological rationale is based on:
- diuresis and inhibition of crystal nucleation;
- the effect of citrate;
- reduction of oxidative stress.

Furthermore, both the quantitative ratios, the preparation method, and the structured dosage regimen demonstrate the actual efficacy of the composition, especially in patients with a high recurrence rate, namely a specific clinical population that has not been considered by any scientific research to date.

## Claims

1. A phytotherapeutic composition for use in the prophylaxis and treatment of reno-ureteral stone disease, comprising a mixture of horsetail plant parts, knotgrass plant parts, and lemon juice.

2. The phytotherapeutic composition for use in the prophylaxis and treatment of reno-ureteral stone disease according to claim 1, wherein said horsetail is selected from *Equisetum hyemale* L., *E. arvense* L., *E. telmateia* Ehrh., *E. giganteum* L., *E. bogotense* Kunth, *E. debile* Roxb. ex Vaucher, *E. myriochaetum*, *E. fluviatile* L., *E. palustre* L., *E. sylvaticum* L., *E. laevigatum*, and *E. ramosissimum* Desf., and said knotgrass is selected from *Polygonum aviculare*, *P. barbatum*, *P. bellardii*, *P. bistorta* L., *P. capitatum*, *P. cognatum*, *P. equisetiforme*, *P. flaccidum*, *P. glabrum*, *P. multiflorum* Thunb., *P. orientale* Linn., *P. polystachyum*, *P. plebeium*, *P. tortuosum*, *P. viviparum*, and *P. hyrcanicum*.

3. The phytotherapeutic composition for use in the prophylaxis and treatment of reno-ureteral stone disease according to claim 1 or 2, wherein said horsetail plant parts are dried leaves and aerial stems, and said knotgrass plant parts are dried leaves, flowers, stems, and roots.

4. The phytotherapeutic composition for use in the prophylaxis and treatment of reno-ureteral stone disease according to any one of claims 1 to 3, consisting of an infusion/tea/decoction comprising, per 1000 ml of water, a weight ratio of horsetail and knotgrass plant parts ranging between 1:2 and 2:1, and lemon juice in an amount between 100 ml and 300 ml.

5. The phytotherapeutic composition for use in the prophylaxis and treatment of reno-ureteral stone disease according to claim 4, wherein said horsetail and knotgrass plant parts are present in an amount ranging between 10 g and 50 g, preferably between 15 g and 30 g.

6. The phytotherapeutic composition for use in the prophylaxis and treatment of reno-ureteral stone disease according to claim 4, wherein the infusion/tea is administered in amounts of 2.5-10 cl, six times daily, before and after the main meals of breakfast, lunch and dinner.

7. The phytotherapeutic composition for use in the prophylaxis and treatment of reno-ureteral stone disease according to claim 5, wherein the infusion is administered for 1-3 months every 3-6 months.

8. The phytotherapeutic composition for use in the prophylaxis and treatment of reno-ureteral stone disease according to any one of claims 1 to 6, for use in the treatment and prophylaxis of reno-ureteral stone disease in "stone former" patients.

9. A process for preparing an infusion/tea/decoction for the prophylaxis and treatment of reno-ureteral stone disease, comprising the steps of:
• providing dried and comminuted horsetail *(Equisetum)* and knotgrass *(Polygonum)* plant parts;
• providing lemon juice;
• boiling water together with said plant parts;
• allowing the mixture to infuse;
• cooling said infusion to room temperature;
• adding said lemon juice.

10. The process according to claim 9, wherein, for every 1000 ml of water to be boiled, said plant parts consist of *Equisetum* leaves and aerial stems and *Polygonum* leaves, flowers, stems and roots, present in amounts ranging between 10 g and 50 g; said lemon juice is obtained by squeezing fresh pulp and is present in an amount of 100-300 ml; and said infusion is allowed to steep for 20-60 minutes.
